# EUROPEAN PATENT APPLICATION

(11) **EP 4 462 444 A1**
(43) Date of publication of application: **13.11.2024**
(21) Application number: 23172745.4
(22) Date of filing: 11.05.2023
(51) Int. Cl.: G16H 50/20, G16H 50/30

(54) **VISUALLY CONVEYING FUTURE STATE OF PHYSIOLOGICAL STATE OF PATIENT**

(71) Applicant: Universität Zürich, 8006 Zürich (CH)
(72) Inventor: TSCHOLL, David Werner, 8053 Zürich (CH); NÖTHIGER, Christoph Beat, 8966 Oberwil-Lieli (CH); MALORGIO, Amos, 8051 Zürich (CH); ROCHE, Tadzio Raoul, 8057 Zürich (CH)
(74) Representative: DeltaPatents B.V.

(57) **Abstract**

A system and method are provided for visually conveying a physiological state of a patient. For that purpose, a graphical representation of a patient may be established on a display. The graphical representation may be configured to visually convey a current state of the physiological parameter using a set of graphical elements. To further visually convey a future state of the physiological parameter using the graphical representation of the patient, a prediction technique may be used to obtain at least one predicted value of the physiological parameter for at least one future time instance and a modified version of a set of graphical elements may be selected for display based on a predicted value of the physiological parameter. The modified version may be modified by a visual attribute of the set of graphical elements having been modified. For example, a rendering style may be modified. This way, a viewer will recognize a visualized future state in the same manner as that the viewer would recognize a visualized current state, while still being able to distinguish the visualization of the future state from that of the current state.

## Description

### FIELD OF THE INVENTION

The invention relates to a system and computer-implemented method for visually conveying a physiological state of a patient. The invention further relates to a patient monitor comprising the system. The invention further relates to a computer-readable medium comprising instructions for a computer program, the computer program comprising instructions to cause a processor system to perform the computer-implemented method.

### BACKGROUND OF THE INVENTION

It is known to monitor physiological parameters of a subject at regular and consecutive time instances. For example, the heartrate of a runner may be monitored to serve as a performance metric during a run. If the monitoring is in the context of medical care, the subject may also be referred to as patient and such monitoring as patient monitoring. In both cases, the measured values of the physiological parameters may be visualized, for example for the user or patient to see (e.g., in self-care scenarios) or for medical personnel to see. Typically, in patient monitoring, the measured values may be compared to static or dynamic thresholds to detect abnormalities in the physical parameters, and if such abnormalities are detected, an alarm may be triggered, e.g., to attract the attention of medical personnel. For example, on an intensive care unit (ICU), a patient's vital signs (heart rate (HR), respiration rate (RR), core body temperature (CBT), oxygen saturation (SpO2) and blood pressure (BP) may be closely monitored and compared to a normal or acceptable physiological range, and the alarm may be triggered if the value of a physiological parameter exceeds or falls below the range. In general, physiological parameters may include, but are not limited to, vital signs such as heart rate, respiration rate, and blood pressure, advanced hemodynamic parameters such as cardiac output, stroke volume, and systemic vascular resistance, hematological parameters, such as red and white blood cell counts and hemoglobin concentration, and analyte parameters, such as glucose or lactate, concentrations in whole blood, plasma, or serum.

It is known in patient monitoring to show a trend view of the measured values of the physiological parameters on a display. Such a trend view may provide a longitudinal visualization of the measurement data by setting out the measured values of the physiological parameters at the consecutive time instances against a common timeline. Typically, in such a trend view, the time is presented on the x-axis while the measured values of the physiological parameters are presented on the y-axis, typically in the form of graphs which are vertically separated from each other along the y-axis but horizontally aligned with respect to the time on the x-axis. Based on the trend view, medical personnel may spot trends in the measured physiological parameters, such as a deterioration in the medical condition of the patient, and intervene in the patient's treatment, if necessary.

However, it may require sufficient time to perceive and understand the information presented in a trend view. Such time is not always available, for example in an emergency situation or when medical personnel have to care for a large number of patients.

It is known to equip a patient monitor with a user interface that enables a user, such as medical personnel, to better perceive and understand the information presented by the patient monitor. For example, US 10702214B2 describes a system and method for visualizing a patient's medical condition, wherein a graphical representation of the patient comprising a body having at least a torso and a head, as well as particularly two legs and two arms, is displayed using a display device, wherein said displayed graphical representation comprises at least one region which is allocated to at least one or several provided (e.g. measured and/or determined) patient monitoring quantities, and wherein the appearance of the at least one region is altered in real-time when the at least one patient monitoring quantity to which said at least one region is allocated changes. In a scientific study [1] in which the graphical representation of the patient was used in clinical practice, it was demonstrated that such a graphical representation of the patient results in a statistically significant improvement in the perception of vital signs of clinical users. In another scientific study [2], it was demonstrated that such a graphical representation of the patient results in a statistically significant improvement in the perception of vital signs with peripheral vision.

The graphical representation of the patient as described in US 10702214B2 addresses at least some of the disadvantages of a trend view. However, the graphical representation concerns a patient's current state. Medical personnel are frequently also concerned with the patient's future state and may attempt to, based on the presented information, mentally identify trends to try to identify whether or not physiological parameters of the patient remain in a normal or acceptable physiological range. Disadvantageously, such mental exercises may also be time-consuming and error-prone. It would therefore be desirable to enable the graphical representation of US 10702214B2, as well as other clinical decision support tools, to also concern themselves with a patient's future state without requiring a user to mentally identify trends on the basis of the presented information.

### References

[1] Tscholl DW, Handschin L, Neubauer P, Weiss M, Seifert B, Spahn DR, Noethiger CB. Using an animated patient avatar to improve perception of vital sign information by anaesthesia professionals. Br J Anaesth. 2018 Sep; 121(3):662-671. doi: 10.1016/j.bja.2018.04.024. Epub 2018 May 24. PMID: 30115265.
[2] Pfarr J, Ganter MT, Spahn DR, Noethiger CB, Tscholl DW. Avatar-Based Patient Monitoring With Peripheral Vision: A Multicenter Comparative Eye-Tracking Study. J Med Internet Res. 2019 Jul 17;21(7):e13041. doi: 10.2196/13041. PMID: 31317870; PMCID: PMC6668297.

### SUMMARY OF THE INVENTION

In a first aspect of the invention, a computer-implemented method is provided for visually conveying a physiological state of a patient, comprising:
- accessing a stream of measured values of at least one physiological parameter of the patient, wherein the measured values are obtained at consecutive time instances;
- establishing a graphical representation of the patient on a display, wherein the graphical representation is configured to visually convey a current state of the physiological parameter using a set of graphical elements, wherein the set of graphical elements is selected from a predetermined number of different sets of graphical elements, which different sets of graphical elements each represent a different state of the physiological parameter, based on a current measured value of the physiological parameter;
   wherein the method further comprises visually conveying a future state of the physiological parameter using the graphical representation of the patient by:
- using a prediction technique to, based on the stream of measured values, generating a prediction of a value of the physiological parameter at at least one future time instance, thereby obtaining at least one predicted value of the physiological parameter;
- providing a modified version of each different set of graphical elements and selecting the modified version of a set of graphical elements for display based on the predicted value of the physiological parameter, wherein the modified version of the set of graphical elements is modified with respect to the set of graphical elements by a visual attribute of the set of graphical elements having been modified.

In a further aspect of the invention, a system is provided for visually conveying a physiological state of a patient, comprising:
- an input interface for accessing a stream of measured values of at least one physiological parameter of the patient, wherein the measured values are obtained at consecutive time instances;
- a display interface to a display;
- a processor subsystem configured to, via the display interface, establish a graphical representation of the patient on the display, wherein the graphical representation is configured to visually convey a current state of the physiological parameter using a set of graphical elements, wherein the set of graphical elements is selected from a predetermined number of different sets of graphical elements, which different sets of graphical elements each represent a different state of the physiological parameter, based on a current measured value of the physiological parameter;
   wherein the processor subsystem is further configured to visually convey a future state of the physiological parameter using the graphical representation of the patient by:
- using a prediction technique to, based on the stream of measured values, generating a prediction of a value of the physiological parameter at at least one future time instance, thereby obtaining at least one predicted value of the physiological parameter;
- providing a modified version of each different set of graphical elements and selecting the modified version of a set of graphical elements for display based on the predicted value of the physiological parameter, wherein the modified version of the set of graphical elements is modified with respect to the set of graphical elements by a visual attribute of the set of graphical elements having been modified.

In a further aspect of the invention, a transitory or non-transitory computer-readable medium is provided, the computer-readable medium comprising data representing a computer program comprising instructions for causing a processor system to perform a computer-implemented method as described in this specification.

The above measures relate to patient monitoring, which may typically involve monitoring the condition of a patient in a clinical setting, e.g., in a care centre, but which may also include non-clinical settings, e.g., in a self-care setting, as well as non-medical settings, e.g., when monitoring the performance of a user during exercise. In such patient monitoring, physiological parameters of the patient may be measured at consecutive time instances, for example at regular intervals, e.g., every 100ms, 1 sec, 1 minute, 10 minutes, etc., or at irregular intervals, e.g., when changes in the physiological parameters are expected to occur. For such measurements, various known types of sensors and/or sensing devices may be used, including but not limited to the types described elsewhere in this specification.

The above measures involve providing a system and a computer-implemented method which have access to the measured values of one or more physiological parameters of a patient, for example by accessing real-time or near real-time data stream comprising the measured values. For example, the system may be connected to a patient monitor or may in some examples be a subsystem of a patient monitor. Another example is that the system may be a workstation or mobile device which has remote access to the measured values of the physiological parameters measured by such a patient monitor. In yet another example, the system may be directly connected to one or more sensors which are arranged for measuring one or more physiological parameters of the patient.

The above measures further involve establishing a graphical representation of the patient on a display. The graphical representation may also be referred to as an 'avatar' in that it may graphically represent a patient by showing a human-like form. For example, the graphical representation may be used as described in US 10702214B2, which is hereby incorporated by reference in respect of the graphical representation and in which the graphical representation of the patient is described to have a body having at least a torso and a head as well as two legs and two arms. The graphical representation may be configured to visually convey a current state of the physiological parameter using a set of graphical elements, with the term `set' referring to 'one or more'. These graphical elements may take any suitable form, such as bitmaps, polygons, voxels, point-clouds, etc., and may be selected from a predetermined number of different sets of graphical elements, in which each of the sets of graphical elements may represent a different state of the physiological parameter. For example, to convey a state of the brain activity of the patient, one set of graphical elements may graphically represent a pair of wide-open eyes to convey too high brain activity, while another set of graphical elements may graphically represent a pair of shut eyes to convey too low brain activity. A set of graphical elements may thus define one or more graphical objects which form part of the graphical representation of the patient. Such graphical objects may, but do not need to, represent recognizable anatomical parts of a patient, such as the aforementioned pair of eyes, a heart, a lung, a thumb, etc.

Typically, only one of the sets of graphical elements for a particular physiological parameter is displayed at any particular time. Namely, the set of graphical elements to be displayed may be selected by the system and method based on a current measured value of the physiological parameter. This way, the current state of the physiological parameter may be conveyed to a viewer, such as a clinician or a nurse. Moreover, when the state of the physiological parameter changes, this may typically be reflected in the current measured value of the physiological parameter, which may cause a change in the displayed set of graphical elements if the change is significant enough. This way, the appearance of a part of the graphical representation of the patient may be dynamically adapted to convey a current state of the physiological parameter of the patient.

In other words, the sets of graphical elements for a particular physiological parameter may define different appearances of a part of the graphical representation of the patient, with each appearance being different from another so as to convey the respective state of the physiological parameter via the current appearance. Such differences may for example lie in the colour, shape, movement (e.g., in case a set of graphical elements defines an animation), etc., of the set of graphical elements, or combination of such aspects. It is further noted that the state of the physiological parameter may represent a categorization of the value of the physiological parameter into a limited number of categories, which such categorization being in the following also referred to as a 'classification'. Specific examples of classes may include 'normal', 'too low', and 'too high'. While the measured values of the physiological parameter may assume many different values, the state of the physiological parameter may thus assume (much) fewer different classes than values, for example 10, 9, 8, 7, 6, 5, 4, 3 or 2 different classes. It is further noted that the classification and classes may in some examples be specific to the type of physiological parameter, in that for different types of physiological parameters, a different classification and classes may be used.

The system and method are further configured to visually convey a future state of the physiological parameter using the graphical representation of the patient. For that purpose, one or more future values of the physiological parameter may be predicted, which future value(s) may be indicative of the future state of the physiological parameter. For example, as also elucidated elsewhere in this specification, a machine learning model may be used to predict the future value(s) from current and past value(s) of the physiological parameter. It is noted that in some examples, the classification which maps the future value to a future state may be the same as for the current value of the physiological parameter, while in other examples, a different classification may be used, for example one which uses fewer classes, or which uses different thresholds or a different classification technique.

To visualize the future state, the different sets of graphical elements which are used to visualize the current state of the physiological parameter may be modified by changing a visual attribute of the graphical elements. The visual attribute which is modified may be one or one of a small subset of the many visual attributes of the graphical elements. These modified versions of the sets of graphical elements may also be referred to as 'modified sets' of graphical elements. In particular, the overall appearance of the graphical object(s) defined by the modified sets of graphical elements, for example in terms of their spatial arrangement in relation to each other and in relation to the graphical representation of the patient, may substantially remain unchanged, while nevertheless introducing a distinct difference between the modified and unmodified sets which is recognizable by a user. Based on the future value(s) of the physiological parameter, one of the modified sets of graphical elements may then be selected and subsequently displayed by the system and method.

The above measures are based on the insight that it is desirable for a viewer, such as a clinician or a nurse, to be able to obtain a prediction of a future state of the physiological parameter of the patient and to be able to distinguish between the visualization of the current state and the future state of the physiological parameter, but without having to learn new visual elements, new numbers, new visualization paradigms, etc. For that purpose, the graphical elements used for the visualization of the current state of the patient are re-used for the visualization of the future state of the patient but modified to a degree which allows the viewer to clearly distinguish whether the visualization pertains to the current state or to the future state. This may be accomplished by modifying one or a small subset of visual attributes of the graphical elements while leaving the majority of visual attributes unchanged. This may allow a viewer who is familiar with the sets of graphical elements which are used to convey the current state of the physiological parameter to effortlessly recognize a future state of the physiological parameter, not only in terms of class (e.g., which state is represented) but also that the state is indeed a future state and not a current state. By way of the above measures, a significant challenge in clinical practice is addressed as the above measures increase the situational awareness of clinical staff through predictive technology but without additionally contributing to the cognitive load of the clinical staff, with would otherwise be undesirable as clinical staff is often already at their cognitive load limits with the information currently already displayed by patient monitors and similar systems.

Optionally, the visual attribute which is modified is a rendering style of the set of graphical elements, for example a line drawing style or line drawing colour, an area filling style or area filling colour, or a transparency. By modifying a rendering style of the set of graphical elements, the overall spatial arrangement and shape of the graphical elements may remain entirely or at least substantially unchanged, thereby allowing the viewer to still recognize a visualized future state in the same manner as that the viewer would recognize a visualized current state. Effectively, the 'what is displayed' may remain unchanged, e.g., in terms of the type of visual objects and their overall shape and position, while only the 'how it is displayed' may be modified. For example, when continuous lines are used for the visualization of a current state, dashed or dotted lines may be used for the visualization of the future state. Another example is that the line thickness, line style, line colour etc., may be modified. Additionally, or alternatively, filled areas of the set of graphical elements may be modified, e.g., by changing their fill pattern or fill colour or fill transparency. Additionally, or alternatively, the overall transparency of the set of graphical elements may be modified, e.g., being non-transparent for the visualization of a current state and partially transparent for the visualization of a future state. Additionally, or alternatively, an additional graphical object may be shown to indicate that the current visualization pertains to the future state rather than to the current state, for example by displaying an aura around the set of graphical elements or around the graphical representation of the patient or by displaying a symbol which is associated with the future or a prediction, e.g., a fortune teller symbol.

Optionally, the method further comprises, and/or the processor subsystem is further configured for:
- determining a confidence of the prediction of the value of the physiological parameter;
- providing the modified version of the set of graphical elements only if the confidence exceeds a confidence threshold.

Predictive technology may suffer from a lack of acceptance and trust by clinical staff as such predictions may not always be correct and it may often not be clear to clinical staff how the predictions are made. For example, when a machine learning model is used for prediction, the machine learning model may be regarded by clinical staff as a 'black box' with unknown contents. To increase acceptance and trust, the visualization of a future state may only be shown when there is sufficient confidence in the prediction so as to build trust with the clinical user. Confidence may be determined in various ways. For example, if a machine learning model is used for prediction, the machine learning model may, in addition to the predicted value, also output a confidence value. This confidence value may then be compared to a threshold to determine whether the confidence in the prediction is sufficiently high. In general, confidence may be determined for the particular predicted value at hand or for the particular setting in which the system and method are operated (e.g., the type of physiological parameter, the prediction horizon for which predictions are made, etc.). The latter type of confidence may be determined based on test data, e.g., in the form of historical data, for which a ground truth is available. For example, this way, it may be determined that the confidence of the prediction technique is high when applied to a first type of physiological parameter but low for a second type of physiological parameter. In such a case, the system and method may visualize predicted future states for the first type of physiological parameter but not for the second type of physiological parameter. Another example is that it may be determined that the confidence is high for a short prediction horizon but low for a long prediction horizon, or vice versa. Also in such a case, future states may only be visualized when the system and method are used in a setting in which there is sufficient confidence that the predictive values are likely to be correct.

Optionally, the confidence of the prediction is determined based on an average of sensitivity and specificity of the prediction technique. The average of sensitivity and specificity may also be referred to as a 'balanced accuracy' and may represent a suitable confidence metric. Both the sensitivity and specificity may be determined based on test data and may be determined for a specific settings (e.g., a type of physiological parameter, a prediction horizon for which predictions are made, etc.) to be able to determine when to provide and when not to provide predictions.

Optionally, the method further comprises, and/or the processor subsystem is further configured for:
- determining if the predicted value of the physiological parameter crosses a physiological threshold associated with the physiological parameter; and
- providing the modified version of the set of graphical elements only if the predicted value of the physiological parameter has crossed the physiological threshold.

The predictions of future states of the physiological parameter may only be provided if the predictions are of particular clinical relevance. This may in general be the case when the predicted value of the physiological parameter crosses a clinically relevant physiological threshold, such as an upper or lower threshold of a normal physiological range associated with the particular type of physiological parameter. This way, future states may be visualized when they represent a deviation from normal.

Optionally, the method further comprises, and/or the processor subsystem is further configured for:
- determining a degree of criticality of the predicted value of the physiological parameter having crossed the physiological threshold; and
- conveying the degree of criticality by selecting at least one of: a further visual attribute, a display time, and a display frequency, of the modified version of the set of graphical elements based on the degree of criticality

In some situations, it may be considered more critical that the predicted value of the physiological parameter has crossed a physiological threshold and it may be desirable to convey this criticality to the viewer. Namely, this way, clinical staff may be made aware of the criticality of the prediction and may be able to timely act upon the prediction, e.g., by intervening in the care of the patient, for example with medication. For example, prediction of a significant worsening of the physiological state of the patient which have a high confidence and are predicted to occur in the near future may be considered to be more critical than predictions of only a minor worsening, having a low confidence, and/or occurring only in the far future. The system may thus determine the criticality, e.g., as two classes (`critical' or 'not critical') or three or more classes (e.g., 'high criticality', 'medium criticality', 'low criticality') and visualize the criticality. For example, critical predictions may be shown for a longer time and/or more frequently than non- or less critical predictions. Another example is that another visual attribute of the set of graphical elements may be modified to convey the criticality. This way, the viewer may be informed of the criticality without having to interpret numerical values or text.

Optionally, the method further comprises, and/or the processor subsystem is further configured for determining the degree of criticality based on at least one of:
- a confidence of the prediction of the value of the physiological parameter;
- a time difference with respect to the future time instance of the prediction; and
- a difference between the predicted value of the physiological parameter and the physiological threshold.

For example, a high confidence prediction may be assigned a higher criticality than a low confidence prediction, a prediction for the near future may be assigned a higher criticality than a prediction for the far future, and/or a prediction which represents a significant deviation from the physiological threshold may be assigned a higher criticality than a prediction which represents a minor deviation from the threshold.

Optionally, the further visual attribute is a transparency or a flashing display of the modified version of the set of graphical elements. A lower criticality prediction may be displayed by displaying the modified set of graphical elements with more transparency, e.g., to indicate a lower confidence in the prediction and/or that the prediction is for the far future, while a higher criticality may be displayed with less or no transparency, e.g., to indicate a higher confidence in the prediction and/or that the prediction is for the near future. Alternatively, or additionally, a flashing display may be used to alert the viewer to a higher criticality of the future state of the physiological parameter, while less or non-critical predictions may be displayed without flashing.

Optionally, the method further comprises, and/or the processor subsystem is further configured for providing the modified version of the set of graphical elements simultaneously with set of graphical elements to simultaneously convey the current state of the physiological parameter and the future state of physiological parameter. Both the current state and the future state may be visualized simultaneously. For example, corresponding sets of graphical elements (e.g., the modified and non-modified versions) may be shown in a co-located manner, e.g., at a same or similar position on screen. If the future state and the current state are the same, the corresponding sets of graphical elements may then practically coincide, which may not, or only to a lesser degree, draw the attention of the viewer, which may be desirable if the state remains unchanged. However, if the future state is different from the current state, a different set of graphical elements may be selected for the future state than for the current state, which may then draw the attention of the viewer as both sets of graphical elements may not coincide.

Optionally, the method further comprises, and/or the processor subsystem is further configured for omitting a numerical visualization of one or more of: the predicted value of the physiological parameter, the future state of the physiological parameter, and the future time instance. In some examples, the prediction may be visualized by way of a modified set of graphical elements while expressly omitting displaying numerical visualizations. In other examples, such numerical visualizations may be displayed in addition to the modified set of graphical elements.

Optionally, the different sets of graphical elements are selected to be visually distinguishable from each other. This way, a viewer may clearly distinguish between the different states represented by the respective sets of graphical elements.

Optionally, the modified version of each set of graphical elements is selected to be visually distinguishable from each respective set of graphical elements.

Optionally, the physiological parameter is one of: temperature, heart rate, pulse rate, ST-segment deviation, invasive arterial blood pressure, central venous pressure, respiratory rate, tidal volume, end-tidal CO2 concentration, train of four ratio, bispectral index, oxygen saturation, and non-invasive arterial blood pressure.

Optionally, the prediction technique is configured to generate the prediction for at least one future time instance within a prediction horizon of one of 10 seconds, 20 seconds, 30 seconds, 1 minute, 2 minutes, 5 minutes, 10 minutes, 20 minutes, 30 minutes, 1 hour, and 2 hours. The prediction horizon may determine how far ahead predictions are provided. A prediction horizon between 10 seconds and 2 hours has been found to be clinically relevant for various types of physiological parameters.

Optionally, a machine learning model may be provided which is configured, by training, to predict, using a first time-series of measured values of the physiological parameter as input, a second time-series of values of the physiological parameter as output, wherein the second time-series represents a numerical prediction of the values of the physiological parameter ahead in time of the first time series. In other words, the machine learning model may be trained to predict a time-series of future values of a physiological parameter of a patient based on a time-series of past measured values of the physiological parameter. The prediction technique may use the machine learning model to generate, using a time-series of measured values from a real-time or near real-time stream as input, a time-series of predicted values as output. This time-series of predicted values may represent a prediction of the value of the physiological parameter for a number of time instances in the future and may be used to select the modified version of a set of graphical elements for display. In other words, instead of basing the selection of which modified set of graphical elements to display only on a single predicted value, a time-series of predicted values may be used. Such a time-series of predicted values may better represent the future state of the physiological parameter and thus allow the future state to be more accurately determined. Depending on the configuration of the machine learning model and the prediction technique, the time-series may span a particular time period in the future, e.g., 40 seconds or 20 minutes or 1 hour, and may have a particular time resolution, e.g., one predicted value (sample) per second or one sample per 10 seconds, per minute, per five minutes, etc.

Optionally, the time-series of predicted values spans a time period selected between seconds and tens of minutes, for example a time period selected between 10 seconds and 2 hours. The time period may also be referred to as a prediction horizon. A prediction horizon between 10 seconds and 2 hours has been found to be clinically relevant for various types of physiological parameters.

Optionally, the time-series of predicted values has a time resolution selected between half a second and minutes, for example comprising predicted values for every second, every 10 seconds, every minute, or every five minutes. Such a time resolution may also be referred to as a sampling rate and may also be expressed in Hz.

Optionally, the prediction technique uses at least two machine learning models to generate predictions, wherein a first machine learning model is configured to make shorter term predictions and a second machine learning model is configured to make longer term predictions, wherein the processor subsystem is configured to combine outputs of the first machine learning model and the second machine learning model to obtain one time-series of classifications to be provided to the clinical decision support tool. Both a short prediction horizon and a long prediction horizon may be clinically relevant. It may be advantageous to provide separate machine learning models for the respective prediction horizons, for example to account for the fact that the training data for such prediction horizons may be different. A short prediction horizon may be for example be advantageous when predicting vital sign deviations for surgical patients undergoing anaesthesia. For example, a hypotension prediction perioperatively becomes significant only when an adverse event is predicted with a very high probability seconds to a few minutes before the event. When such an adverse event is predicted, it is possible to prepare for and quickly counteract hypotension when it arises. However, it is unlikely that an anaesthesiologist would give a patient a vasopressor 20 minutes in advance when hypotension is forecasted in 20 minutes. In other words, an anaesthesiologist may be reluctant to act for predictions far in the future, but may still be interested in having such predictions, e.g., to be able to identify a longer-term trend. Another advantage of having a machine learning model with a short prediction horizon is that such a model may be quicker to start outputting predictions as it may have a lower start-up latency. In clinical use, this means that such a model may need less time to start outputting predictions when a data stream from a new patient is arriving in real-time.

Optionally, the first machine learning model is configured to output a first time-series of predicted values which has a first time resolution selected between half a second and tens of seconds, and wherein the second machine learning model is configured to output a second time-series of predicted values which has a second time resolution selected between tens of seconds and minutes. Here, the term 'tens of seconds' may refer to any one of: 10, 20, 30, 40 and 50 seconds.

Optionally, the machine learning model is trained to predict the time-series of values of the physiological parameter using a further time series of values of a further physiological parameter as further input. The past measured values of a second physiological parameter may also be predictive for the future values of the first physiological parameter. Accordingly, the machine learning model may be trained to use the past measured values of both types of physiological parameters to provide a prediction of future values of the first physiological parameter. Advantageously, a more accurate prediction of the future values of the first physiological parameter is obtained.

Optionally, the machine learning model is one of: recurrent neural network, such as a long short-term memory neural network, a logistic regression-based model, and a decision-tree based model.

It will be appreciated by those skilled in the art that two or more of the above-mentioned embodiments, implementations, and/or optional aspects of the invention may be combined in any way deemed useful.

Modifications and variations of any of the systems, any of the computer-implemented methods and/or any of the computer programs, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a system configured to access a stream of measured values of a physiological parameter of a patient and to visually convey a physiological state of the physiological parameter using a displayed graphical representation of a patient;
Fig. 2 shows a display output of the system in which a display area shows measured physiological parameters and in which a graphical representation of a patient is dynamically adapted to visually convey the current physiological state of the patient;
Fig. 3A shows several measured physiological parameters;
Figs. 3B and 3C shows two time-series of the measured values of a physiological parameter being selected for the training of a machine learning model, wherein the first time-series of measured values is selected as input and the second time-series of measured values, which is ahead in time with respect to the first time-series, is selected as a training target;
Fig. 4A shows a time-series of predicted values of a physiological parameter, wherein the time-series is obtained by applying the machine learning model to a time-series of measured values of the physiological parameter;
Fig. 4B shows a comparison of the time-series of predicted values of the physiological parameter against a physiological range define by an upper threshold and a lower threshold;
Fig. 4C shows a time-series of classifications which are indicative of a respective predicted value falling inside, above or below the physiological range.
Figs. 5A-5C show different animation phases of a graphical representation of a patient which conveys a predicted future state of arterial pressure;
Figs. 6A-6C show different animation phases of a graphical representation of a patient which conveys predicted future states of end-tidal CO2 and tidal volume;
Fig. 7 shows a graphical representation of a patient which conveys predicted future states of brain activity, relaxation, and central venous pressure;
Fig. 8 shows a graphical representation of a patient which conveys a predicted future state of arterial pressure;
Fig. 9 shows a method of visually conveying a physiological state of a patient;
Fig. 10 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 10: physiological parameter
- 12: data stream of measured values of physiological parameter

- 100: system for conveying physiological state of patient
- 120: sensor interface
- 140: processor subsystem
- 160: memory
- 180: display interface
- 182: display data
- 190: display

- 200, 202: display area comprising measured physiological parameters
- 210: time axis
- 212: value axis
- 220: data stream of measured values of physiological parameter
- 222: first time-series of measured values
- 224: second time-series of measured values
- 230: numerical visualization of current measured value
- 240: time series of predicted values
- 250: predicted value
- 260: physiological range
- 262: lower threshold
- 264: upper threshold
- 270: classification with respect to physiological range

- 300: graphical representation of patient
- 310: graphical element(s) for arterial pressure
- 320: graphical element(s) for central venous pressure
- 330: graphical element(s) for end-tidal CO2
- 340: graphical element(s) for tidal volume
- 350: graphical element(s) for heart rate
- 360: graphical element(s) for brain activity
- 370: graphical element(s) for relaxation

- 410-416: modified graphical element(s) for arterial pressure
- 420: modified graphical element(s) for central venous pressure
- 430-432: modified graphical element(s) for end-tidal CO2
- 440-444: modified graphical element(s) for tidal volume
- 460: modified graphical element(s) for brain activity
- 470: modified graphical element(s) for relaxation

- 500: method of visually conveying physiological state of patient
- 510: accessing data stream of measured values of physiological parameter
- 520: establishing graphical representation of patient on display to visually convey current state of physiological parameter
- 530: visually conveying future state of physiological parameter using graphical representation
- 540: predicting future value of physiological parameter
- 550: displaying modified version of set of graphical elements to visually convey future state of physiological parameter

- 600: non-transitory computer-readable medium
- 610: data representing computer program

### DETAILED DESCRIPTION OF EMBODIMENTS

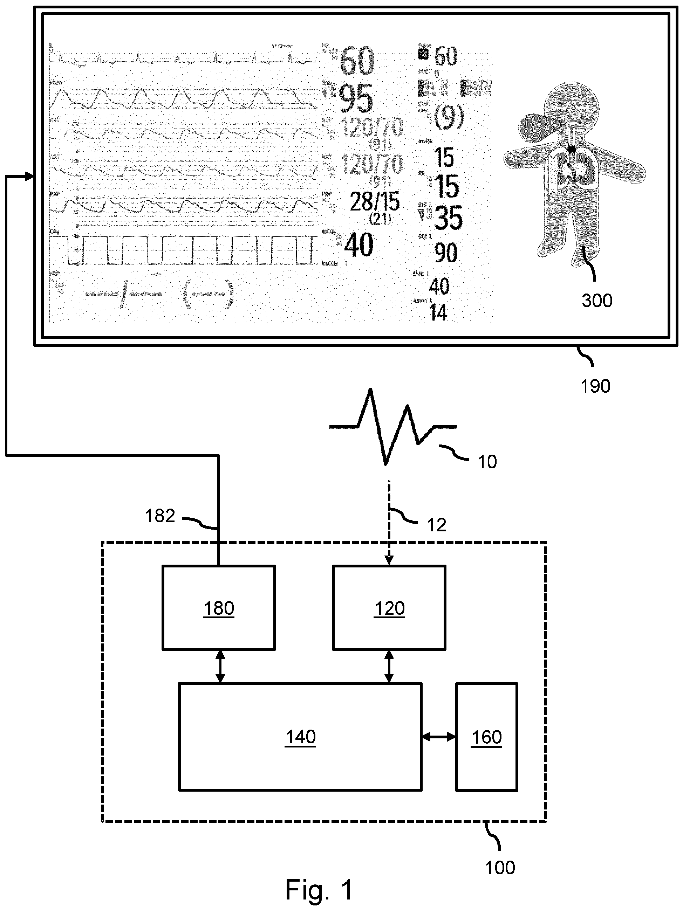

**Fig. 1** shows a system 100 for visually conveying a physiological state of a patient. The system 100 may comprise an input interface 120 configured to access a data stream 12 of measured values of a physiological parameter 10 of the patient. Such a data stream 12 may for example be received in real-time or near real-time and may for example be received from a patient monitor (not shown in Fig. 1) which is connected to one or more sensors to measure physiological signals of the patient, or directly from one or more of such sensors. Examples of sensors include, but are not limited to, a Swan Ganz pulmonary artery catheter (PAC), Philips PiCCO (which combines an arterial line and a central venous line), Edwards ClearSight (which uses finger cuffs), Edwards FloTrac (using an arterial line), echography, Philips ShellCuff (using a cuff around the upper arm), etc. The input interface 120 may for example be a network interface such as an ethernet interface or a bus-type interface such as an USB interface. In some examples, the system 100 may be part of a patient monitor, in which case the input interface 120 may be an internal interface.

The system 100 may further comprise a memory 160. The memory 160 may serve as shorter term data storage for the system 100. For example, the data stream 12 received via the input interface 120 may be buffered, that is at least temporarily stored, in the memory 160. Another example is that instruction data may be stored in the memory 160 for execution by a processor subsystem of the system 100. The memory 160 may for example be a volatile memory or a persistent memory, e.g., in form of a flash memory. Although not shown in Fig. 1, the system 100 may also comprise a further data storage which may be used for longer term storage. Such a data storage may take any suitable form, such as a hard disk or a solid-state disk or an array of hard disks or an array of solid-state disks. It is noted that the further data storage may also be used for shorter term data storage, for example to buffer the data stream 12 received via the input interface 120.

The system 100 may further comprise a display interface 180 to a display 190 to enable the system 100 to visualize output of the system 100 via the display 190. In the example of Fig. 1, the display is shown to be an external display 190. Alternatively, the display may be an internal display of the system 100. Although not shown in Fig. 1, the system 100 may further comprise a user interface subsystem which may be configured to, during operation of the system 100, enable a user to interact with the system 100, for example using a graphical user interface shown on the display 190. For that purpose, the user interface subsystem may comprise a user input interface configured to receive user input data from a user input device operable by the user. The user input device may take various forms, including but not limited to a computer mouse, touch screen, keyboard, microphone, etc. In general, the user input interface may be of a type which corresponds to the type of user input device, e.g., a thereto corresponding type of user device interface.

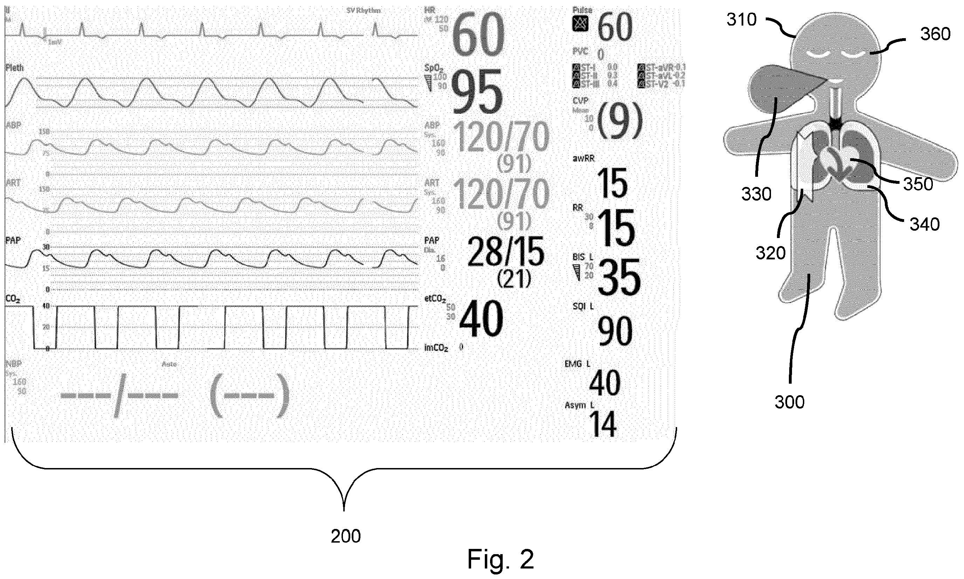

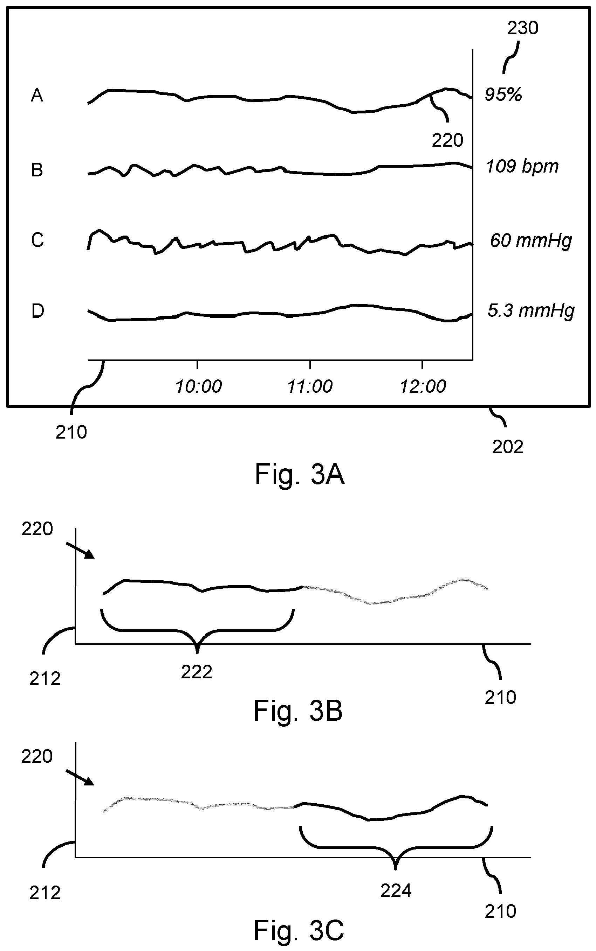

The system 100 is further shown to comprise a processor subsystem 140 which may be configured to internally communicate with the input interface 120, the memory 160, and the display interface 180. The processor subsystem 140 may be configured, for example by instruction data stored in the memory 160, to establish a graphical representation 300 of the patient on the display 190. As will also be further explained with reference to Fig. 2, the processor subsystem 140 may be configured to visually convey a current state of a physiological parameter using the graphical representation 300 and in particular using a set of graphical elements which are part of the graphical representation of the patient. For that purpose, the processor subsystem 140 may select the set of graphical elements from a predetermined number of different sets of graphical elements, which different sets of graphical elements may each represent a different state of the physiological parameter. This selection may be performed based on a current measured value of the physiological parameter, meaning that the current measured value may determine which set of graphical elements is selected by the processor subsystem 140. As will also be further explained with reference to Figs. 3A-8, the processor subsystem 140 may be further configured to visually convey a future state of the physiological parameter using the graphical representation of the patient. For that purpose, the processor subsystem 140 may use a prediction technique to, based on the data stream 12, generate a prediction of a value of the physiological parameter at at least one future time instance, thereby obtaining at least one predicted value of the physiological parameter, and provide a modified version of each different set of graphical elements and select the modified version of a set of graphical elements for display based on the predicted value of the physiological parameter. The modified version of the set of graphical elements may be modified with respect to a corresponding set of graphical elements by a visual attribute of the set of graphical elements having been modified. These and other operations of the system 100, and various optional aspects thereof, will be explained in more detail elsewhere in this specification.

In general, the system 100 may be embodied as, or in, a single device or apparatus. The device or apparatus may be a general-purpose device or apparatus, such as a workstation or a computer, but may also be an application-specific device or apparatus, such as a patient monitor. The device or apparatus may comprise one or more microprocessors which may represent the processor subsystem, and which may execute appropriate software. The software may have been downloaded and/or stored in a corresponding memory, e.g., a volatile memory such as RAM or a non-volatile memory such as Flash. Alternatively, functional units of the system, e.g., the input interface, the display interface, and/or the processor subsystem, may be implemented in the device or apparatus in the form of programmable logic, e.g., as a Field-Programmable Gate Array (FPGA). In general, each functional unit of the system 100 may be implemented in the form of a circuit. It is noted that the system 100 may also be implemented in a distributed manner, e.g., involving different devices or apparatuses. For example, the distribution may be in accordance with a client-server model, e.g., using a server and workstation.

**Fig. 2** shows a display output of the system 100 of Fig. 1 in more detail. The display output is shown to comprise a display area 200 which shows measured physiological parameters which may be received by the system 100, or which may be measured by the system 100 in examples where the system 100 is (part of) a patient monitor. Such display of measured physiological parameters is also further explained with reference to Fig. 3A. The display output is further shown to comprise the aforementioned graphical representation 300 of a patient. Generally speaking, this graphical representation 300 may be dynamically adapted by the system 100 to visually convey a current physiological state of the patient to a viewer. For that purpose, the graphical representation 300, which may in the following also be referred to as a 'visual patient avatar', may comprise respective parts which may be adapted to convey the physiological state of different physiological parameters of the patient. Each of these parts may be represented by a set of graphical elements, which in turn may be selected from different sets of graphical elements which may be provided to visualize different physiological states of a respective physiological parameter. The selection of which set of graphical elements is to be displayed may be based on the current value of a respective physiological parameter. In this respect, it is noted that 'current' may refer to a recently or most recently received measured value, but which measured value may have been measured in the past, e.g., seconds or minutes or longer ago. The adjective 'current' thus does not imply that the measurement has been performed at exactly the current time.

In the example of Figs. 1 and 2, the visual patient avatar 300 is shown to comprise respective parts 310-360, each being represented by a respective set of graphical elements, to convey the physiological state of respectively arterial pressure 310, central venous pressure 320, end-tidal CO2 330, tidal volume 340, heart rate 350, and brain activity 360. Each part may be adapted in its appearance by a different set of graphical elements being selected, being for example different in terms of colour, shape, location, movement, etc. Examples for the visualization of the aforementioned physiological states are given in table 1 below. A respective set of graphical elements may also define an animation, with the animation being different for the respective sets of graphical elements for the respective physiological parameter. In general, the different sets of graphical elements for a respective physiological parameter may be generated so as to be visually distinguishable from each other by a human viewer. For example, when the different sets of graphical elements define animations for physiological states of a physiological parameter, the respective animations may be played-out by the system 100 at different speeds, e.g., at different rendering frequencies, so as to convey the difference in physiological state. For example, the avatar's lungs may be animated to resemble breathing with a slow, normal, or high frequency. The difference between the rendering frequencies may be selected to be perceptible by a viewer.

In general, while the physiological parameter may assume a value in a continuous range, only a limited number of physiological states may be defined to represent the measured value of the physiological parameter. For example, the physiological states may correspond to 'within physiological range', 'above physiological range' and 'below physiological range'. The physiological range may be used-definable and may be used by clinical staff to define what is clinically considered a normal physiological range associated with the physiological parameter. Alternatively, or additionally, the physiological range may be predefined or may have a default value which may be overridden. In general, the physiological state may be determined based on one or more thresholds defined for the physiological parameter. Accordingly, a different set of graphical elements may be selected if a physiological parameter has crossed a threshold. Two of such thresholds may be used to define a physiological range as described above. Alternatively, only a single threshold may be used, or more than two thresholds. In general, a crossing of a threshold may be considered to represent a transition from one physiological state to another.

Example of physiological parameters, physiological states, and visualizations in the visual patient avatar are shown in table 1 below. Here, qualifications such as 'slow', 'quick', 'low', 'high', 'normal', etc., may represent thresholds which are set, e.g., by a user or by default, to values which are clinically considered to represent such qualifications.

**Table 1: Visualisations of physiological parameters**

| **Physiological parameter** | **Visualisation in the avatar** | **Phenomenon in a real patient after which the visualisation is modelled** |
|---|---|---|
| 1. Pulse rate | The pulsation frequency of the body of the avatar. | The pulse wave synchronous pulsation of the arteries, as visible, for example, under a microscope or palpable as a pulse. |
| | Example: The avatar's body pulsates with a slow, normal, or quick frequency. | |
| 2. Arterial blood pressure | The pulsation intensity of the body of the avatar. | The strength of the pulse in the arteries depending on the arterial blood pressure, as visible, for example, under a microscope or palpable as a pulse. |
| | Example: The avatar's body pulsates just barely, normally (e.g., reaching the white line designating the normal pulsation extension) or very intensely, extending far beyond the white "normal" line. | |
| 3. Central venous pressure | The area of the vena cava of the avatar | The filling volume of the vena cava dependent on central venous pressure. |
| | Example: The size of the avatar's vena cava is very small, normal (e.g., reaching the white line designating the normal extension) or very large, far beyond the white "normal-line". | |
| 4. Respiratory rate | The breathing frequency of the lungs of the avatar and the corresponding exhalation frequency of the CO2 cloud. | The breathing synchronous volume change of the lungs and the breathing synchronous, invisible exhalation of a volume of CO2. |
| | Example: The avatar's lungs breathe with a slow, normal, or high frequency and (if data from a CO2 sensor is available) synchronous slow, normal, or quick exhalation of the CO2 cloud. | |
| 5. Tidal volume | The extension size of the lungs of the avatar during the breathing cycle. | The volume change of the lungs depending on tidal volume. |
| | Example: The avatar's lungs extend just barely, normally (e.g., reaching the white line designating the normal breathing extension) or very far, noticeably beyond the white "normal-line". | |
| 6. Expiratory CO2 concentration | The extension size of the CO2 cloud of the avatar during the breathing cycle. | The volume change of CO2 exhaled into the air. |
| | Example: The CO2 cloud is just barely visible, reaches a normal extension (e.g., reaching the white line designating the normal breathing extension) or extends very wide, far beyond the white "normal" line. | |
| 7. Body temperature | The presence or absence of temperature indicators on the body of the avatar. | Hyperthermia: The heat radiation from the skin. |
| | Example: Heat waves are rising from the avatar, or ice crystals are visible on its skin. | Hypothermia: The skin cold to the touch. |
| 8. Brain activity | The form of the eyes of the avatar. | High: Eyes open. Pupils middle wide, as in sympathetic activation. |
| | Example: The eyes of the avatar are open or closed. | Low: Eyelids completely closed, as in a sleeping patient. |
| 9. Peripheral oxygen saturation | The colour of the body of the avatar. | Normal: light brown skin colour according to Fitzpatrick skin type III. |
| | Example: The avatar has a healthy skin colour or a purple skin colour. | Hypoxia: dark purple skin colour as in cyanosis. |
| 10. Neuromuscular function | The form of the body of the avatar. | Normal neuromuscular function: Extended extremities and thumb (healthy muscle tone in the adductor policis muscle, where care providers frequently measure relaxation). Neuromuscular block: floppy limbs. |
| | Example: The avatar has extended extremities and an extended thumb, or the extremities appear floppy. | |
| 11. Electrocardiography ST-Segment | The presence or absence of a hypoxia indicator over the heart of the avatar. | Normal: the light red colour of healthy myocardium. |
| | Example: The heart muscle of the avatar has a homogenous vivid red colour or a dark purple colour. | Hypoxia: the purple colour of hypoxic myocardium. |

**Figs. 3A-4C** show an example of how to generate a prediction for a value of a physiological parameter, which are also described in a co-pending patent application EP23163213.4 and hereby incorporated by reference in as far as generating a prediction of a value of a physiological parameter. In this example, a time-series of predicted values is generated. However, this is not a limitation, in that a prediction technique may also generate a predictive value for one future time instance at a time. In addition, while the following refers to the use of a machine learning model to generate the prediction, any other prediction technique may be used instead, for example one which is not based on machine learning.

**Fig. 3A** shows an example of measurements of physiological parameters 220 which may be displayed by a patient monitor in a display area 200 of a display. In this example, the measurements of the physiological parameters 220 are displayed along a horizontal axis 210 along which time is set out and which axis may also be referred to as time axis, and a vertical axis along which the measured values of the physiological parameters are set out, being in this example from top to bottom the blood oxygen saturation, mean arterial pressure, the heart rate, and the central venous pressure. Typically, during operation of the patient monitor, the current or most recently measured values of the physiological parameters are plotted at the right-hand side of the display area 200 while past measured values are over time moved toward the left-hand side. Typically, the current or most recent measured values of the physiological parameters are numerically shown 230 at the right-hand side of the display area 200, showing that the current or most recent measured values are respectively "95%", "60 mmHg", "109 bpm", and "5.3 mmHg".

As elucidated elsewhere in this specification, it may be desirable to be able to provide a prediction for a physiological parameter of a patient for use by a clinical decision support tool. In some examples, the clinical decision support may also be implemented by a patient monitor, for example the patient monitor showing a graphical representation of the patient which visually conveys a current state of the patient, while in other examples, the clinical decision support tool may be implemented outside of a patient monitor.

To obtain a prediction for the physiological parameter, a machine learning model may be trained. As input to the training, training data may be used, which training data may comprise recordings of real-time or near real-time the data streams of measured values of a physiological parameter of one or more patients. For example, one or more of the data streams visualized by the patient monitor as shown in Fig. 3A may be recorded and used for training purposes. In some examples, the data streams of several patients may be used. In some examples, the training data may comprise data from several care centres, for example from several hospitals. The training may comprise several iterations, with an iteration involving training the machine learning model on a particular part of the training data. In some examples, the training may pass through the training data several times, e.g., in respective epochs.

**Figs. 3B and 3C** illustrates the data selection for an iteration of the training, with Fig. 3B showing a first time-series 222 of values of the physiological parameter having been selected from the data stream 220. This first time-series may represent a subset, e.g., a segment, selected from the data stream, being in this example a data stream of measured blood oxygen values. For example, the first time-series may be comprised of tens of samples, with each sample representing a measured value. The samples may be selected as consecutive samples from the data stream, but may in some examples also be selected by subsampling the data stream. For example, every second, third, fourth, fifth, 10^{th}, etc. sample from the data stream may be selected. The first time-series 222 may serve as input to the machine learning model during the training iteration. In addition, a second time-series may be selected to serve as prediction target in the training iteration. As shown in Fig. 3C, the second time-series 224 may be selected as a set of samples which are ahead in time of the first time-series 222. For example, the second time-series 224 may immediately follow the first time-series 222, or in some examples may be minorly spaced apart in time.

Having selected the first time-series 222 and the second time-series 224 of measured values from the data stream 220, the machine learning model may be trained with the first time-series 222 being used as input to the machine learning model and the second time-series 224 as prediction target. Accordingly, the machine learning model may be trained to predict the second time-series 224 using the first time-series 222 as input. Thereby, the machine learning model may be trained to predict a time-series of future values of the physiological parameter, being in this example the blood oxygen saturation, based on a time-series of current and/or past measured values. The training process may continue by selecting, in a next iteration of the training, another time-series of measured values as input and yet another time-series of measured values as prediction target, which process may repeat itself for a number of iterations. In some embodiments, the time-series which are selected as input during the training may be selected not to overlap in time, meaning that each iteration in an epoch of the training uses a different part of the training data as input.

With continued reference to the selection of the first time-series and the second time-series, it is noted that the length of the first time-series and the second time-series, e.g., in number of samples, may be the same, for example 20 samples each. However, this is not a limitation, in that the lengths may also be selected differently. For example, the length of the second time-series may be selected to be equal to or shorter than the length of the first time-series. For example, 20 samples may be used as input to the machine learning model to train the model to predict 10 samples ahead in the future. In some examples, the second time-series may consist of a single predicted sample.

In some specific examples, the machine learning model may be trained for one of the following prediction tasks (which may also be considered regression tasks by the prediction involving the prediction of continuous values): prediction of 10, 20, and 40 seconds ahead (using 1-sec data, e.g., a sample rate of 1 Hz) and 5, 10, 20, 30, 60, 90, 180 minutes ahead (using 1-min data, e.g., a sample rate of 1/60 Hz). Accordingly, the machine learning model may, when trained to predict 20 minutes ahead, receive 20 samples covering 20 minutes of the data stream as input and may predict, from this input, the values of the physiological parameter as 20 samples covering the next 20 minutes. In other examples, two or more machine learning models may be trained, each having a different prediction horizon, referring to the time period which is predicted ahead, and/or having a different time resolution, referring to the time by which individual samples are spaced apart in time. For example, a first machine learning model may be trained to predict 40 seconds ahead with a sampling rate of 1 Hz, meaning that the first machine learning model may provide an array of 40 samples as output, while a second machine learning model may be trained to predict 20 minutes ahead with a sampling rate of 1/60 Hz (e.g., 1 minute), meaning that the second machine learning model may provide an array of 20 samples as output. The outputs of both machine learning models may be combined as described elsewhere in this specification.

The machine learning model may take any suitable form. For example, the machine learning model may be a neural network, such as a recurrent neural network. In a specific example, the neural network may be a deep recurrent neural network which uses a long-short-term-memory layer which processes the input data, e.g., the samples of the first time-series, and computes an inner representation of the input data. The neural network may further comprise a dense layer as output layer to compute the prediction, e.g., the samples of the second time-series. The size of the dense layer may be selected in accordance with the prediction horizon, for example equal the number of samples to be predicted. For example, for a prediction of 20 minutes ahead with a sampling rate of 1 sample per minute, a dense layer of size 20 may be used. In other examples, the machine learning model may be a logistic regression-based model, or a decision-tree based model.

Having trained the machine learning model, the trained machine learning model may be made accessible to the system as described elsewhere in this specification. The system may use the trained machine learning model to provide a prediction for a physiological parameter of a patient and output the prediction to a clinical decision support tool. This may involve applying the trained machine learning model to a time-series of measured values from a real-time or near real-time data stream as input, for example on a continuous or periodical basis. For example, the data stream may be a stream of measured blood oxygen saturation values as shown in Figs. 3A-3C. The time-series of measured values may be selected to comprise a current or most recent measured value and a number of preceding measured values. In general, the time period and/or the time resolution of the series of measured values may be selected to, but does not need to, correspond to the time period and/or the time resolution of the respective first time-series which are used as input during the training of the machine learning model.

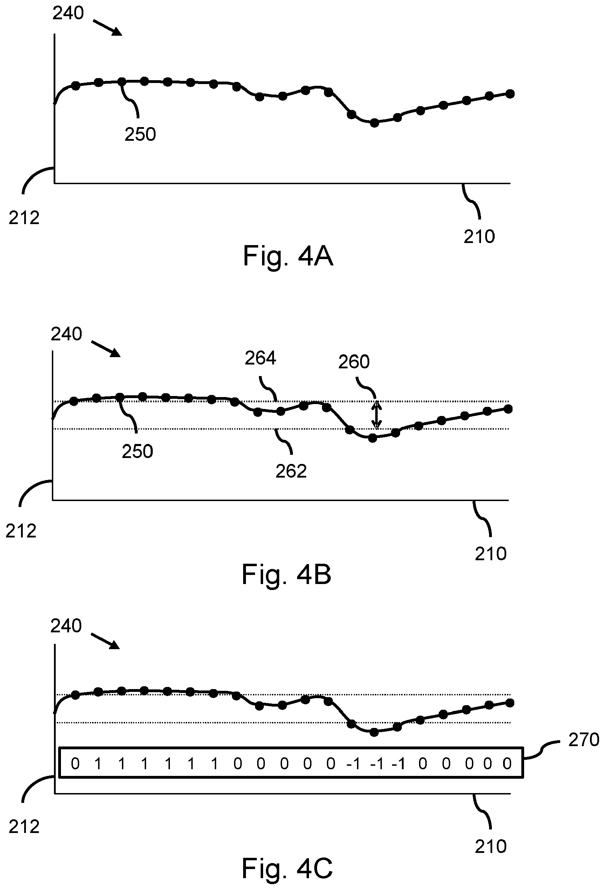

**Fig. 4A** shows a time-series of predicted values 240 of a physiological parameter, obtained by applying the machine learning model to the aforementioned time-series of measured values of the physiological parameter. The time-series 240 is shown to be comprised of 20 individual samples 250, which may for example cover a shorter time period of 20 seconds or a longer time period of 20 minutes or 1 hour. It is noted that although Figs. 4A-4C show the predicted values 240 as a continuous graph, the continuous graph is merely generated for illustrative purposes by interpolation of the individual samples 250. This also applies to Figs. 3A-3C where likewise continuous graphs are shown for illustrative purposes.

Instead of directly using the time-series of predicted values to adapt the visual patient avatar, the system may first determine for the time-series of predicted values whether the predicted values fall within a physiological range associated with the physiological parameter, thereby obtaining a time-series of classifications, wherein a respective classification is indicative of whether a predicted value falls inside or outside of the physiological range, and in some examples, whether the predicted value exceeds an upper threshold of the range or whether the predicted value falls below a lower threshold of the range. Such a physiological range may for example correspond to a normal or acceptable physiological range and may in some examples be user-definable, for example as user-definable parameters which are accessible via a graphical user interface.

**Fig. 4B** shows this comparison of the time-series of predicted values of the physiological parameter against a physiological range 260 defined by an upper threshold 262 and a lower threshold 264. It can be seen that some of the samples 250 exceed the upper threshold 262, that other samples fall below the lower threshold 264, and that yet other samples remain within the physiological range 260. **Fig. 4C** shows a time-series of classifications 270 which may be generated by comparison with the physiological threshold, and which are indicative of a respective predicted value falling inside, above or below the physiological range. This time-series of classifications 270 may represent a time-series of quantifications of the physiological state of the physiological parameter and may be used by system to adapt the visual patient avatar. In this example, the classification has resulted in numerical class labels having been assigned a value in a ternary classification system, e.g., '0' for falling within the physiological range, '1' for exceeding the upper limit, and `-1' for falling below the lower limit. These classifications may be considered to directly represent physiological states of the physiological parameter, for example with a transition to '0' causing the system to display a set of graphical elements representing a 'normal' state of a physiological parameter, a transition to '1' causing the system to display a set of graphical elements representing an 'abnormal - too high' state of the physiological parameter, and a transition to `-1' causing the system to display a set of graphical elements representing an 'abnormal - too low' state of the physiological parameter. However, it will be appreciated that any other suitable type of classification and corresponding physiological state may be used instead, for example using other numerical labels, using non-numerical labels, using more or fewer classes, etc. An example of the use of fewer classes may be the assignment of a class label and corresponding state of '0' for falling within the physiological range and '1' for falling outside of the physiological range, or vice versa. It is noted that in general, the physiological state may not need to directly correspond to a classification with respect to a threshold. For example, the time-series of classifications 270 may first be temporally filtered to filter-out incidental crossings of a threshold. This may avoid spurious state changes and corresponding spurious changes in the appearance of the visual patient avatar.

It is noted that the system and method may make use of two or more machine learning models. For example, a first machine learning model may be trained to make shorter term predictions and a second machine learning model may be trained to make longer term predictions. In a specific example, the first machine learning model may have a prediction horizon up to 1 minute and the second machine learning model may have a prediction horizon above 1 minute. In a yet more specific example, the first machine learning model may have a prediction horizon of 40 seconds and a time resolution of 1 sample per second and the second machine learning model may have a prediction horizon of 20 minutes and a time resolution of 1 sample per minute. In another specific example, the second machine learning model may have a prediction horizon of 100 minutes and a time resolution of 1 sample per 5 minutes. The predictions of both models may be combined into a single time-series of predicted values, e.g., by concatenation, and then classified as described above with reference to Figs. 4A-4C. It is noted that both model outputs may also be individually classified, and their resulting time-series of classifications may be combined, e.g., by concatenation, into a single time-series of classifications to be used to adapt the appearance of the visual patient avatar. Alternatively, the system and method may keep the outputs of both models separately and separate sets of graphical elements may be used to separately visualize the respective shorter term predictions and the longer term predictions.

The same techniques described with reference to Fig. 2 and elsewhere for the visualization of a current value of a measured physiological parameter may be used to visualize the predicted value (which may also be referred to as 'future value') of the physiological parameter, except that the respective sets of graphical elements may be modified to visually signify that a respective visualization is a prediction. This may for example involve modifying a rendering style of the set of graphical elements, for example a line drawing style or line drawing colour, an area filling style or area filling colour, or a transparency. This way, a viewer may be able to visually distinguish between a visualization of a current physiological state of a physiological parameter and a visualization of a predicted physiological state of the physiological parameter. Both types of visualizations may be provided simultaneously, for example to allow a viewer to continue to monitor the current physiological state of the physiological parameter. However, as also explained elsewhere in this specification, the visualization of a predicted physiological state of the physiological parameter may be provided intermittently, for example only if the physiological state is predicted to deteriorate in the near future and if this prediction is considered to be accurate.

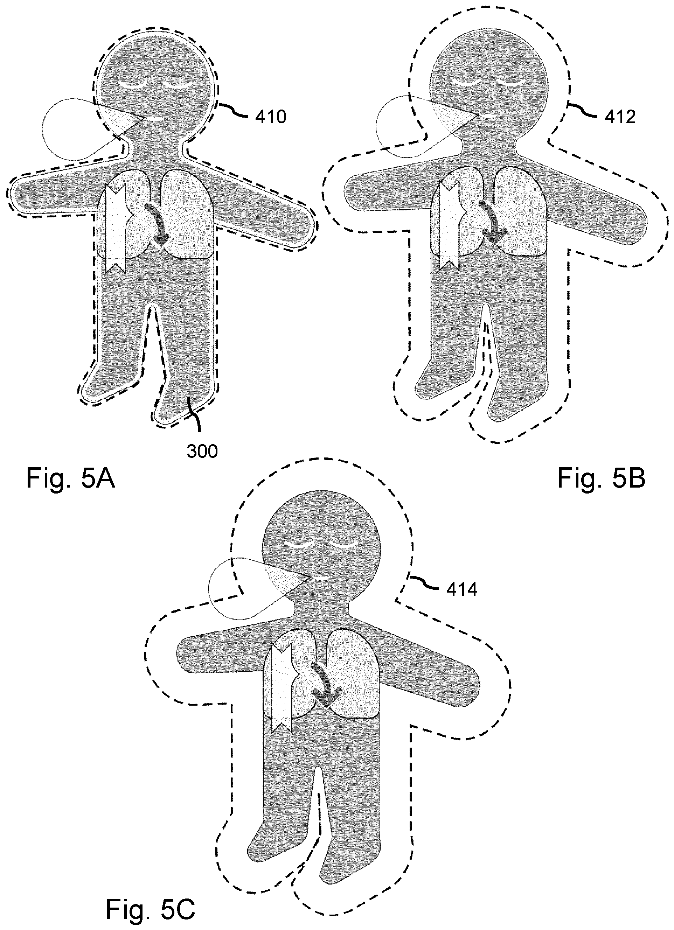

**Figs. 5A-5C** show a graphical representation 300 of a patient which conveys a predicted future state of arterial pressure through a pulsation of an outline 410-414 of the body of the visual patient avatar. This visualization, by way of a set of graphical elements, may define an animation having five consecutive phases which may be repeated over time, with Fig. 5A showing phases 1 and 5, Fig. 5B showing phases 2 and 5, and Fig. 5C showing phase 3. The animation phases may together, e.g., as a sequence of phases 1, 2, 3, 4, and 5, define the pulsation of the outline 410-414 of the body, with the extent of the outline relative to the body's reference contour (e.g., how far the outlines 410-414 deviate from the reference contour) indicating the predicted state of the blood pressure. For example, Figs. 5A-5C may represent a visualization for an arterial pressure which is predicted to be 'too high' (rather than, e.g., `normal' or 'too low'). To specifically show that the state of the arterial pressure is a predicted future state of the arterial pressure, the outline may be shown as a dashed line, while for the current state, the outline may be shown as a continuous line.

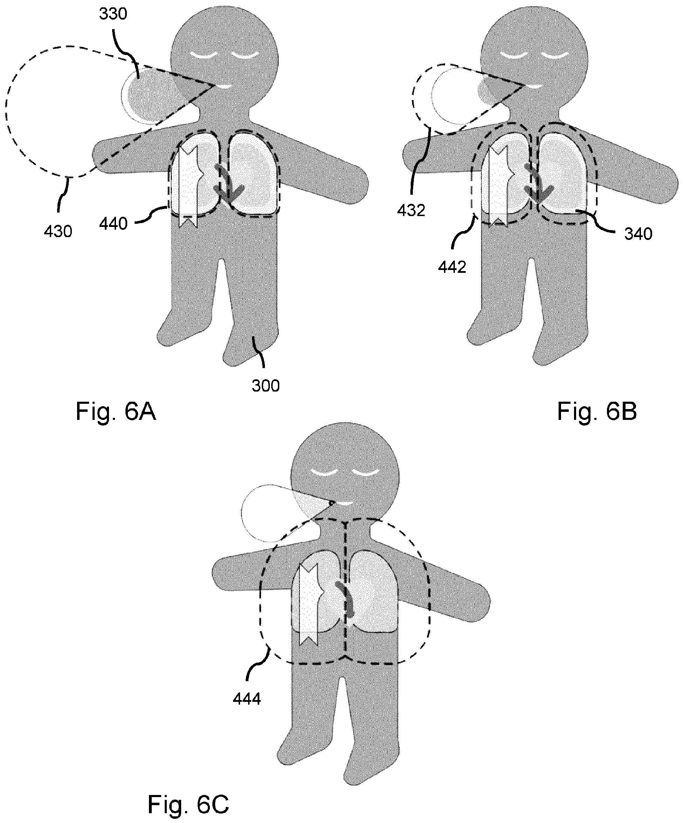

**Figs. 6A-6C** show a graphical representation 300 of a patient which conveys the predicted future state of end-tidal CO2 and tidal volume. As in the case of Figs. 5A-5C, the respective figures show different animation phases defined by a set of graphical elements, with Fig. 6A showing phases 1 and 5, Fig. 6B showing phases 2 and 4, and Fig. 6C showing phase 3. The tidal volume may be visualized by an outline 440-444 of the lung, with the extent of the lung relative to the lung's reference contour (e g., how far the outlines 410-414 deviate from the reference contour) indicating the predicted state of the tidal volume. For example, Figs. 6A-6C may represent a visualization for a tidal volume which is predicted to be 'too high' (rather than, e.g., `normal' or 'too low'). As in the case of Figs. 5A-5C, to specifically show that the state of the tidal volume is a predicted future state of the tidal volume, the outline may be shown as a dashed line, while for the current state, the outline may be shown as a continuous line. In Figs. 6A-6C, the current state 340 is shown simultaneously with the predicted state and may represent a 'normal' state.

With continued reference to Figs. 6A-6C, another set of graphical elements may represent the end-tidal CO2 concentration. More specifically, the end-tidal CO2 concentration may be visualized by the size of a CO2 cloud 430, 432, and Figs. 6A-6C may represent a visualization for an end-tidal CO2 concentration which is predicted to be 'too high' (rather than, e.g., `normal' or 'too low'). Again, to specifically show that the state of the tidal volume is a predicted future state of the tidal volume, the outline may be shown as a dashed line, while for the current state, the outline may be shown as a continuous line. In the example of Figs. 6A-6C, also the current state 330 of the end-tidal CO2 concentration is shown simultaneously with the predicted state and may represent a 'normal' state.

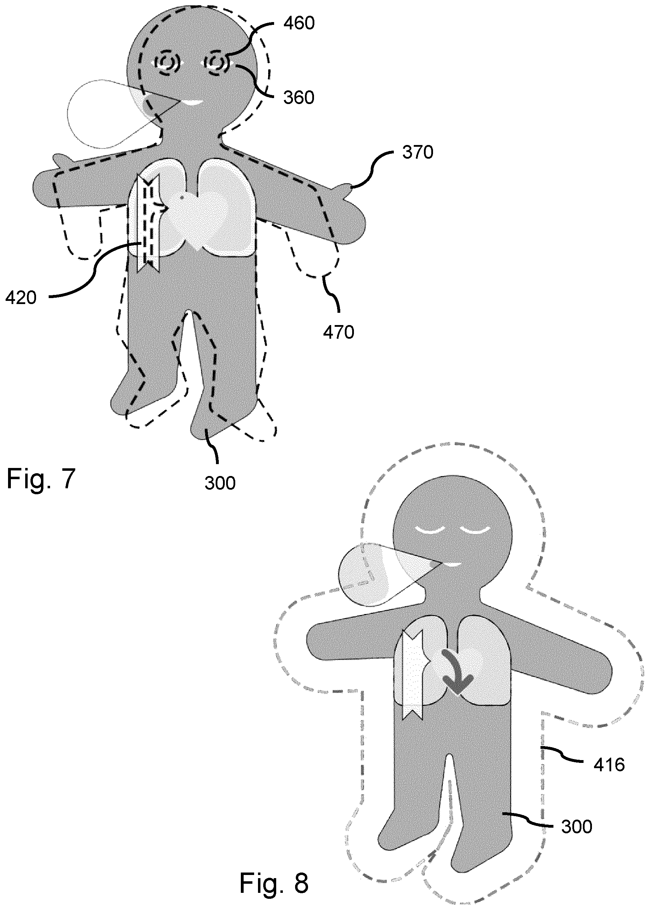

**Fig. 7** shows a graphical representation 300 of a patient which conveys the predicted future states of brain activity, relaxation (neuromuscular function) and central venous pressure. The current brain activity may be visualized by a set of eyes 360 which are shown to be closed to convey 'low' brain activity. The brain activity is predicted to be 'high' in the future, which may be visualized by a set of eyes 460 shown with dashed lines and being fully open with middle-wide pupils. The current relaxation state, which may be 'normal', may be visualized by the visual patient avatar having extended extremities and an extended thumb 370, while the patient may be predicted to have muscle relaxation which is visualized by the extremities 470 of the body appearing floppy. Moreover, the central venous pressure may be predicted to be 'low' and visualized by the small size of the avatar's vena cava 420.

**Fig. 8** shows a graphical representation 300 of a patient which conveys a predicted future state of arterial pressure. This visualization is similar to that of Fig. 5C but shows an alternative rendering style of the visualization of future predictions, in that the line pattern 416 is shown to alternate in colour/grey value to denote the future prediction.

In general, the visualization of a predicted physiological state of the physiological parameter may be displayed for a predetermined time period and in some cases repeatedly. The frequency of repetition may also be referred to as a 'display frequency' and may indicate how often per time unit a predictive visualization is shown. The time period and/or the display frequency may be selected based on a criticality of a prediction. This criticality may in the following and elsewhere also be referred to as 'urgency'. In one specific example, three different urgencies may be distinguished: urgent, intermediate, and non-urgent. In case of an urgent prediction, a corresponding visualization may be displayed for one time unit every two time units and may be flashing during the display. A non-urgent prediction may be shown for one time unit every eight time units and may be made partially transparent. An intermediate urgency prediction may be shown for one time unit every four time units and may not be flashing nor partially transparent during the display. In another specific example, a time unit may be selected as a value between 1 seconds and 20 seconds, or between 2 seconds and 10 seconds, or between 2.5 seconds and 5 seconds. A very specific example of a time unit is 3.5 seconds. In another specific example, the display frequency may be increased if a prediction pertains to the near future. For example, if a physiological parameter is predicted to fall below the lower threshold of a normal range in 20 minutes, its predictive visualisation may be displayed less frequently per time unit than if the vital sign were to reach the lower threshold already in 30 seconds.

In some examples, the graphical representation may show predictive visualizations only if there is sufficient confidence in the prediction. For example, as confidence measure, the so-called balanced accuracy may be used which corresponds to an average between sensitivity and specificity values (e.g., (*sensitivity* + *specificity*)/2) and, hence, may serve as a single confidence measure. The graphical representation of the patient may be configured to show predictive visualizations only for those physiological parameters for which the balanced accuracy is expected to be greater than a threshold, for example larger 0.9. This functionality may be explained to a user that whenever a prediction is displayed, the probability of the prediction occurring is more than 90 out of 100.

In some embodiments, the future time instance to which a prediction pertains (e.g., a change from `normal' to 'too high' in 12 minutes) may not be displayed to a viewer. As such, there may be no explicit indication of when an event is predicted to occur. However, since the prediction may be continuously updated, the visualization of a prediction may disappear when no longer valid, for example because an intervention has been made. As such, if a viewer continues to see the prediction, e.g., either continuously or repeatedly, the viewer may be and remain aware that the prediction is still valid. In some embodiments, predictive visualizations may only be provided up-to a maximum prediction horizon, for example up-to 5 minutes, 10 minutes, 20 minutes, etc. In such a case, the system may not make predictions, or may not visualize the predictions, above this prediction horizon.

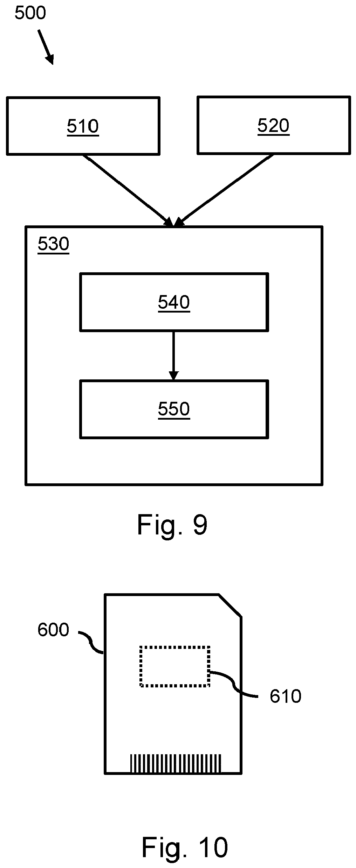

**Fig. 9** shows a block-diagram of computer-implemented method 500 for visually conveying a physiological state of a patient. The method 500 may correspond to an operation of the system 100 of Fig. 1. However, this is not a limitation, in that the computer-implemented method 500 may also be performed using another system, apparatus or device. The method 500 is shown to comprise, in a step titled "ACCESSING DATA STREAM OF MEASURED VALUES OF PHYSIOLOGICAL PARAMETER", accessing 510 a stream of measured values of at least one physiological parameter of the patient as described elsewhere in this specification, and in a step titled "ESTABLISHING GRAPHICAL REPRESENTATION OF PATIENT ON DISPLAY TO VISUALLY CONVEY CURRENT STATE OF PHYSIOLOGICAL PARAMETER", establishing 520 a graphical representation of the patient on a display as described elsewhere in this specification. The method 500 is further shown to comprise, in a step titled "VISUALLY CONVEYING FUTURE STATE OF PHYSIOLOGICAL PARAMETER USING GRAPHICAL REPRESENTATION", visually conveying 530 a future state of the physiological parameter using the graphical representation of the patient by, in a sub-step titled "PREDICTING FUTURE VALUE OF PHYSIOLOGICAL PARAMETER", using a prediction technique to, based on the stream of measured values, generating 540 a prediction of a value of the physiological parameter at at least one future time instance, thereby obtaining at least one predicted value of the physiological parameter, and in a sub-step titled "DISPLAYING MODIFIED VERSION OF SET OF GRAPHICAL ELEMENTS TO VISUALLY CONVEY FUTURE STATE OF PHYSIOLOGICAL PARAMETER", providing 550 a modified version of each different set of graphical elements and selecting the modified version of a set of graphical elements for display based on the predicted value of the physiological parameter, wherein the modified version of the set of graphical elements is modified with respect to the set of graphical elements by a visual attribute of the set of graphical elements having been modified.

It will be appreciated that the steps of the method 500 may be performed in any suitable order, e.g., consecutively, simultaneously, or a combination thereof, subject to, where applicable, a particular order being necessitated, e.g., by input/output relations.

The method may be implemented on a computer as a computer implemented method, as dedicated hardware, or as a combination of both. As also illustrated in **Fig. 10****,** instructions for the computer, e.g., executable code, may be stored on a computer readable medium 600, e.g., in the form of a series 610 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 10 shows a memory device 600.

Examples, embodiments or optional features, whether indicated as nonlimiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A computer-implemented method (500) for visually conveying a physiological state of a patient, comprising:
- accessing (510) a stream of measured values of at least one physiological parameter of the patient, wherein the measured values are obtained at consecutive time instances;
- establishing (520) a graphical representation (300) of the patient on a display, wherein the graphical representation is configured to visually convey a current state of the physiological parameter using a set of graphical elements (310-370), wherein the set of graphical elements is selected from a predetermined number of different sets of graphical elements, which different sets of graphical elements each represent a different state of the physiological parameter, based on a current measured value of the physiological parameter;
wherein the method further comprises visually conveying (530) a future state of the physiological parameter using the graphical representation of the patient by:
- using a prediction technique to, based on the stream of measured values, generating (540) a prediction of a value of the physiological parameter at at least one future time instance, thereby obtaining at least one predicted value of the physiological parameter;
- providing (550) a modified version (410-470) of each different set of graphical elements and selecting the modified version of a set of graphical elements for display based on the predicted value of the physiological parameter, wherein the modified version of the set of graphical elements is modified with respect to the set of graphical elements by a visual attribute of the set of graphical elements having been modified.

2. The method (500) according to claim 1, wherein the visual attribute which is modified is a rendering style of the set of graphical elements, for example a line drawing style or line drawing colour, an area filling style or area filling colour, or a transparency.

3. The method (500) according to any one of claims 1 to 2, further comprising:
- determining a confidence of the prediction of the value of the physiological parameter;
- providing the modified version of the set of graphical elements only if the confidence exceeds a confidence threshold.

4. The method (500) according to claim 3, wherein the confidence of the prediction is determined based on an average of sensitivity and specificity of the prediction technique.

5. The method (500) according to any one of claims 1 to 4, further comprising:
- determining if the predicted value of the physiological parameter crosses a physiological threshold (262, 264) associated with the physiological parameter; and
- providing the modified version of the set of graphical elements only if the predicted value of the physiological parameter has crossed the physiological threshold.

6. The method (500) according to claim 5, further comprising:
- determining a degree of criticality of the predicted value of the physiological parameter having crossed the physiological threshold (262, 264); and
- conveying the degree of criticality by selecting at least one of: a further visual attribute, a display time, and a display frequency, of the modified version of the set of graphical elements based on the degree of criticality.

7. The method (500) according to claim 6, further comprising determining the degree of criticality based on at least one of:
- a confidence of the prediction of the value of the physiological parameter;
- a time difference with respect to the future time instance of the prediction; and
- a difference between the predicted value of the physiological parameter and the physiological threshold.

8. The method (500) according to claim 6 or 7, wherein the further visual attribute is a transparency or a flashing display of the modified version of the set of graphical elements.

9. The method (500) according to any one of claims 1 to 8, further comprising providing the modified version of the set of graphical elements simultaneously with set of graphical elements to simultaneously convey the current state of the physiological parameter and the future state of physiological parameter.

10. The method (500) according to any one of claims 1 to 9, further comprising omitting a numerical visualization of one or more of: the predicted value of the physiological parameter, the future state of the physiological parameter, and the future time instance.

11. The method (500) according to any one of claims 1 to 10, wherein:
- the different sets of graphical elements are selected to be visually distinguishable from each other; and/or
- the modified version of each set of graphical elements is selected to be visually distinguishable from each respective set of graphical elements.

12. The method (500) according to any one of claims 1 to 11, wherein the physiological parameter is one of: temperature, heart rate, pulse rate, ST-segment deviation, invasive arterial blood pressure, central venous pressure, respiratory rate, tidal volume, end-tidal CO2 concentration, train of four ratio, bispectral index, oxygen saturation, and non-invasive arterial blood pressure.

13. A transitory or non-transitory computer-readable medium (600) comprising data (610) representing a computer program, the computer program comprising instructions for causing a processor system to perform the method according to any one of claims 1 to 13.

14. A system (100) for visually conveying a physiological state of a patient, comprising:
- an input interface (120) for accessing a stream (12) of measured values of at least one physiological parameter of the patient, wherein the measured values are obtained at consecutive time instances;
- a display interface (180) to a display (190);
- a processor subsystem (140) configured to, via the display interface, establish a graphical representation (300) of the patient on the display, wherein the graphical representation is configured to visually convey a current state of the physiological parameter using a set of graphical elements (310-370), wherein the set of graphical elements is selected from a predetermined number of different sets of graphical elements, which different sets of graphical elements each represent a different state of the physiological parameter, based on a current measured value of the physiological parameter;
wherein the processor subsystem is further configured to visually convey a future state of the physiological parameter using the graphical representation of the patient by:
- using a prediction technique to, based on the stream of measured values, generating a prediction (250) of a value of the physiological parameter at at least one future time instance, thereby obtaining at least one predicted value of the physiological parameter;
- providing a modified version (410-470) of each different set of graphical elements and selecting the modified version of a set of graphical elements for display based on the predicted value of the physiological parameter, wherein the modified version of the set of graphical elements is modified with respect to the set of graphical elements by a visual attribute of the set of graphical elements having been modified.

15. A patient monitoring system comprising the system (100) according to claim 14.
